# EUROPÄISCHE PATENTANMELDUNG

(11) **EP 1 262 855 A2**
(43) Veröffentlichungstag der Anmeldung: **04.12.2002**
(21) Anmeldenummer: 02010954.2
(22) Anmeldetag: 16.05.2002
(51) Int. Cl.: G06F 1/00

(54) **Sabotagesichere und zensurresistente persönliche elektronische Gesundheitsakte**

(30) Priorität: 29.05.2001 DE 10126138
(71) Anmelder: SIEMENS AKTIENGESELLSCHAFT, 80333 München (DE)
(72) Erfinder: Kleinschmidt, Peter, 91058 Erlangen (DE)

(57) **Zusammenfassung**

Gesicherte elektronische Gesundheitsakte zur Verwaltung aller gesundheitsrelevanten Daten, einschließlich früherer Diagnosen und Behandlungen, eines Patienten in Form von Datenkapseln auf mehreren dezentralen Servern eines Netzes mit einem vom Patienten freigebbaren Zugriffscode, wobei bei jeder Änderung oder Ergänzung einer aufgerufenen Datenkapsel die alten Datenkapsel im Netz gelöscht werden und ein neuer Zugriffscode gebildet wird, unter dem die geänderte Datenkapsel wieder neu im Netz gespeichert wird.

## Beschreibung

Die Erfindung bezieht sich auf eine gesicherte elektronische Gesundheitsakte zur Verwaltung aller gesundheitsrelevanten Daten, einschließlich früherer Diagnosen und Behandlungen, eines Patienten in Form von Datenkapseln auf mehreren dezentralen Servern eines Netzes mit einem vom Patienten freigebbaren Zugriffscode.

Für die aktuelle Behandlung eines Patienten ist es für den Behandelnden extrem wichtig, auf möglichst vollständige Daten über die Krankenvorgeschichte und patientenspezifische Daten, wie Impfungen, Allergien, Unverträglichkeiten usw. zugreifen zu können. Vollständigkeit bedeutet dabei nicht unbedingt hohe Detaillierung, siehe später. Andererseits sind diese Daten sensibel und dürfen nicht in falsche Hände gelangen. Neben seinem Gedächtnis verwendet der behandelnde Arzt Aufzeichnungen in Form einer Patientenakte und schreibt bei der Überweisung an einen anderen Arzt die wichtigsten Daten in ein Überweisungsschreiben. In der Praxis wird daraus ein Problem, wenn der Patient unvorhergesehen an einen neuen Arzt gerät, der aus Zeit- oder anderen Gründen keine Möglichkeit hat, an die Daten seiner Kollegen zu gelangen. Im Übrigen stehen diese Daten dem Patienten nur beschränkt zur Verfügung, was künftig zum technischen und juristischen Problem werden könnte, wenn dem Patienten verschiedene Gesundheitsdienste im Netz angeboten werden.

Bisher gibt es bereits zahlreiche Vorschläge und Testinstallationen, die mittels elektronischer Kommunikationsmittel dieses Problem zu lösen versuchen. Sie basieren zum einen auf einer persönlich bei sich zu tragenden Patientenakte, zum Beispiel in Form einer elektronischen Chipkarte oder zum anderen auf einem zentralen Netzserver, auf den jeder Arzt zugreifen können soll. Die reine Kartenlösung, die bereits seit Jahren diskutiert wird und in einigen Ländern eingeführt ist, hat dabei die Problematik, dass zum einen die Datenmenge nur begrenzt ist, dass keine Verfügbarkeit der Daten für Teledienste gegeben ist, dass sie nur mechanisch in mobile Computing integrierbar ist und dass keine Eingabemöglichkeit durch Tastatur, durch Barcodes oder elektronische Tags zur Verfügung steht.

Die vorstehend angesprochene zentrale Patientenakte wird von Netzbefürwortern immer wieder propagiert. Dabei ergibt sich zum einen die Schwierigkeit, dass ohne einheitliche Daten-Normen eine solche Patientenakte praktisch undurchführbar ist. Darüber hinaus ergeben sich aber auch juristische Probleme zur Datenverwendung, aufwändige Maßnahmen für eine letztlich doch nicht garantierbare Sicherheit und dadurch die Gefahr eines Verlustes der Daten durch Sabotage sowie des Missbrauchs der Daten. Auch eine bereits probeweise eingeführte Einstellung privater Akten bei Providern im Internet kann das angesprochene Problem nicht lösen, da sowohl eine unkontrollierbare Datenweitergabe zu befürchten ist, die Privatheit der Daten nicht garantiert ist und die Daten auch in vielen Fällen untereinander inkompatibel sind.

Die fehlende Sicherheit gilt selbst für Gesundheitsakten der eingangs genannten Art, bei der die gesundheitsrelevanten Daten in Form von Datenkapseln auf mehreren dezentralen Servern eines Netzes mit einem vom Patienten freigebbaren Zugriffscode gespeichert sind, wie dies beispielsweise in der WO 01/18631 A1 vorgeschlagen wird. Wenn nur ein einziges Mal der Zugriffscode in falsche Hände gelangt, so ist auch bei diesem ansonsten relativ sicheren System nach der WO 01/18631 A1 ein fortwährender Missbrauch der Daten nicht zu verhindern.

Der Erfindung liegt daher die Aufgabe zugrunde, eine gesicherte elektronische Gesundheitsakte zu schaffen, die sabotagesicher und zensurresistent ist und eine erhöhte Sicherheit gegen unbefugte Weitergabe oder unbefugte Benutzung der Daten beinhaltet.

Zur Lösung dieser Aufgabe ist erfindungsgemäß vorgesehen, dass bei jeder Änderung oder Ergänzung einer aufgerufenen Datenkapsel die alten Datenkapsel im Netz gelöscht werden und ein neuer Zugriffscode gebildet wird, unter dem die geänderte Datenkapsel wieder neu im Netz gespeichert wird.

Durch diese automatische Änderung des Zugriffscodes bei einer Änderung oder Ergänzung der Datenkapsel hat ein Unbefugter, der aus irgendeinem Weg einmal den Zugriffscode erhalten hat - beispielsweise mit der Berechtigung eines einmaligen Ansehens bestimmter Daten - zwar die Möglichkeit, genau diese Daten immer wieder anzusehen, solange die Datenkapsel nicht geändert worden ist. Mit jeder Änderung der Datenkapsel erfolgt aber zwangsläufig eine Änderung des Zugriffscodes mit Abspeicherung der geänderten Datenkapseln unter diesem neuen Zugriffscode und gleichzeitig die Löschung der alten Datenkapseln. Es ist also für einen Unbefugten im Besitz des alten Zugriffscodes selbst der Zugriff auf diese alten Daten nur sehr begrenzt möglich, da diese bei der ersten Änderung der Datenkapseln alle gelöscht werden.

Der Zugriffscode, der aus persönlichen Daten und Speicherdaten nach Art eines Hash-Keys ausgebildet sein kann, soll in Ausgestaltung der Erfindung eine speziell gesicherte Änderungsberechtigung enthalten, über die das automatische Löschen der alten Datenkapseln bewirkt wird. Damit kann erreicht werden, dass der Berechtigte Dritten Unterzugangsberechtigung erteil, bei denen der Zugriffscode die Änderungsberechtigung nicht enthält, sodass dieser Dritte eine Datenkapsel zwar aufrufen und ansehen, sie aber nicht verändern kann.

In weiterer Ausgestaltung kann dabei auch vorgesehen sein, dass bereits ein Ansehen der Daten aus einer Datenkapsel über ein hiermit postuliertes Logfile, das jeden Zugriff mit Zeitstempel protokolliert, eine Änderung darstellt, die eine automatische Änderung des Zugriffscodes bewirkt. Dies kann aber nur bei einem Ansehen der Daten durch einen Berechtigten mit gleichzeitiger Änderungsberechtigung zweckmäßig sein, da ansonsten das zugelassene Ansehen der Daten durch einen Dritten über die Löschung der alten Datenkapseln und die Abspeicherung der neuen Datenkapseln mit geänderten Zugriffscodes diese Datenkapseln sogar für den eigentlichen Inhaber nicht mal auffindbar machen würde.

Das Löschen der Datenkapseln und das später folgende Neuschreiben sorgt für eine bessere Auslasten der Ressourcen eines freenets und erhöht die Redundanz der im freenet abgespeicherten Datenkapseln, da über längere Zeit hinweg die Gefahr in einem freenet besteht, dass einige beteiligte Peers sich von diesem Netz loslösen und dabei eine oder mehrere Kopien einer Datenkapsel verloren geht.

Die Daten sollen dabei bevorzugt in Form von hier sogenannten Datenkapseln mit gegebenenfalls unterschiedlichen Zugriffcodes im Speichernetzwerk gespeichert sein, wobei dieses Speichernetzwerk ein überall verfügbares Netzwerk nach Art des Internet sein soll, in dem gegebenenfalls ein zensurresistentes Extranetz wie das sogenannten "freenet" zur Speicherung der Daten ausgebildet sein kann. Dieses "freenet" kann im Internet durch eine zertifizierte Software jedem zur Verfügung gestellt werden, wobei diese zertifizierte Software garantiert, dass sie außer den beschriebenen Funktionen keine Hintertüren besitzt, die den illegalen Zugriff zu den Daten ermöglichen könnte.

Das angesprochene Extranetz im Internet kann dabei so ausgebildet sein, dass die Daten-Kapseln selbstorganisiert an unterschiedliche Server weitervermittelt und mehrfach identisch abgespeichert werden, sodass sich dabei möglicherweise auftretende Spuren verlieren und nicht zurückverfolgbar sind.

Darüber hinaus hat diese mehrfache Abspeicherung - wobei der Patient durch Parameterisierung eines Zählers die Zahl der identischen Sicherheitskopien bestimmen kann - den Vorteil, dass der zufällige Absturz eines Speichers, der eine der anonymisierten Daten-Kapseln der elektronischen Gesundheitsakte enthält, nicht zu einem Verlust dieser Daten führt, da ja die Mehrzahl der Sicherungskopien - selbst nach mehrfacher Verteilung im Speichernetz - nicht auf dem gleichen Server abgelegt sein kann.

Unabhängig davon, dass eine solche Daten-Kapsel ja sowieso nur mithilfe des beliebig kompliziert aufbaubaren Zugangscodes gelesen werden kann, den nur der Patient hat und den er dritten , wie Ärzten, Dienstleistern, Krankenkassen oder dergleichen nur in Ausnahmefällen und darüber hinaus möglicherweise auch nur im beschränkten Umfang zur Verfügung stellt, kann zur zusätzlichen Absicherung noch vorgesehen sein, dass die Daten verschlüsselt gespeichert sind, wobei zur Verschlüsselung einer Kapsel bevorzugt ein asymmetrischer Schlüssel verwendet wird mit einem öffentlichen Schlüssel des Patienten zum Verschlüsseln der Patientenakte und einem privaten Schlüssel des Patienten zum Entschlüsseln, wobei der private Schlüssel oder das Schlüsselpaar weiterer Bestandteil der persönlichen Berechtigungsinformation, also des persönlichen Zugriffscodes für das Lesen des Inhalts einer Datenkapsel darstellen.

Gemäß einem weiteren Merkmal der vorliegenden Erfindung kann vorgesehen sein, dass die Inhalte der Datenkapseln durch spezielle Unter-Zugriffscodes von entsprechend autorisierten Dritten, zum Beispiel Ärzten, Dienstleistern, Pharmafirmen, Krankenkassen oder dergleichen in begrenztem Umfang lesbar sind, wobei hierfür bevorzugt Zugangseinrichtungen vorgesehen sind, die es ermöglichen, bestimmte Teile der Daten als Statistikdaten zu extrahieren, zu ergänzen, zu kombinieren und zu schematisieren.

Die anonymisierten Statistikdaten sollen dabei zur weiteren Verwendung, insbesondere für den Abruf durch Pharmafirmen oder Krankenkassen, die hierfür dem autorisierenden Patienten gewisse Vorteile oder Vergütungen zukommen lassen, in - auf Veranlassung des Patienten - spezielle Statistik-Kapseln eingegeben und gespeichert werden, die mit einer global geltenden Kapseladresse versehen sind. Es bedarf also keiner Freigabe des eigentlichen Zugriffscodes zu allen Daten der persönlichen Gesundheitsakte des Patienten um diese Statistikfunktionen mit erfüllen zu können.

Der oder die Zugriffscodes können dabei gemäß einem weiteren Merkmal der vorliegenden Erfindung in spezielle, vorzugsweise tragbare Zugangsgeräte, implementiert sein, wie beispielsweise eine Chipkarte, ein Handy, eine Uhr, ein Amulett oder dergleichen, sie können aber auch in einen öffentlichen Zugangsgegenstand, also beispielsweise ein Netzportal oder dergleichen eingegeben werden. Das Zugangsgerät kann dabei in an sich bekannter Weise durch ein Authentifizierungssystem gesichert sein, wie beispielsweise durch eine PIN-Nummer, um bei Verlust des Zugangsgerätes einem Missbrauch vorzubeugen.

Um einen vollständigen Datenverlust im Falle eines Verlustes einer Kapseladresse zu vermeiden, kann in weiterer Ausgestaltung der Erfindung auch vorgesehen sein, dass zumindest Teile der Patientenakten in Ablagevorrichtungen bei den Ärzten, Dienstleitern oder dergleichen, gegebenenfalls auch nur teilweise für diese lesbar, gespeichert sind, die für den Patienten zugänglich sind, um im Falle des Verlustes einer Kapseladresse eine Rekonstruktion einer neuen Daten-Kapsel aus diesen Kopien zu ermöglichen.

Die wichtigen Gesundheitsinformationen, die in einer erfindungsgemäßen sabotagegesicherten und zensurresistenten persönlichen elektronischen Gesundheitsakte sicher und doch für vielfältige Gesundheitsanwendungen abrufbar gespeichert sein sollen, umfassen zum einen langfristige, im Interesse des Patienten geheimzuhaltenden Informationen, also alle jene historischen bis aktuellen Daten sowie Spekulationen und Ratschläge, die für eine jedwede zukünftige Beratung oder Behandlung als bedeutungsvoll erachtet werden. Dazu gehören Anamnese, Befunde, Abschlussberichte sowie Belege Medizinischer Stadien, wie Fotos, diagnostische Bilder, Videos und Tondokumente. Hypothesen, Zwischenschritte, Irrwege, negative Befunde und so weiter sind nur in ihrem Ergebnis und gemäß ihrer voraussichtlichen zukünftigen Bedeutungen, nicht aber in allen Einzelheiten zu vermerken. Dabei kann ein Teil dieser Daten direkt auf dem persönlichen Zugangsgerät zusätzlich zu den persönlichen Berechtigungsinformationen lokalisiert sein (z. B. Notfalldaten) und/oder als Zeiger, das heißt als spezielle Adresse, ausgebildet sein, über den man ohne Barrieren direkt über das überall verfügbare Netzwerk mithilfe dessen die erfindungsgemäße Gesundheitsakte realisiert wird - zum derzeitigen Zeitpunkt wäre dies speziell das sogenannte Internet - an diese Daten gelangt.

Zum anderen handelt es sich um kurzfristige vertrauliche Daten, wie Behandlungsdaten, Verschreibungen, Messwerte, Beobachtungen, Ratschläge usw., die nach einiger Zeit ausgewertet oder erledigt sind und gelöscht werden. Die daraus resultierenden Daten werden in angemessenen Abständen zum Bestand der langfristigen Daten hinzugefügt. Für kurzfristige und langfristige Daten können dabei - wie es bereits weiter oben vorgeschlagen worden ist - unterschiedliche Kapseln mit unterschiedlichen Hash-Adressen verwendet werden, wobei beide Hash-Adressen mithilfe ein und desselben individuellen Zugangsgeräts oder auch mit unterschiedlichen, voneinander getrennten Zugangsgeräten erreicht werden können. Die Auswahl erfolgt im ersteren Fall mittels Bediensoftware oder mittels einer Konfigurationsmöglichkeit auf dem individuellen Zugangsgerät.

Zusammenfassend ist also festzuhalten, dass die erfindungsgemäße elektronische Gesundheitsakte durch Datenstrukturen gekennzeichnet ist, sodass die Daten nur in dem Umfang gelesen werden können, wie der Nutzer Rechte dazu dem Patienten gegenüber ausweisen kann. Der Patient kann selbst auch alle Teile der Akten lesen, sofern er auf einen psychologischen Schutz vor schockierenden Daten verzichtet und hat auch Bereiche in denen er schreiben, also Daten verändern, kann. Die bekannte Professional-Card erlaubt den Ärzten ebenfalls nur Zugriffe auf bestimmte Teile. Aufgrund doppelter (mehrfacher) Verschlüsselung bleiben ihm aber Teile unlesbar (sogenanntes Rollenkonzept). Der Patient kann auch mehrere Kapseln definieren und entscheiden, zu welchen er wem Zugang gewährt. Das Rollenkonzept kann über Schlüssel oder andere Zugriffsbeschränkungen realisiert werden.

Weitere Vorteile, Merkmale und Einzelheiten der Erfindung ergeben sich aus der weiteren Beschreibung einiger Ausführungsbeispiele sowie anhand der Zeichnung. Dabei zeigen:
- Fig. 1: ein schematisches Ablaufdiagramm des Zugriffs eines Berechtigten auf im freenet gespeicherte Datenkapseln und des Löschens der alten Datenkapseln im freenet,
- Fig. 2: die Veränderung der Daten der auf dem lokalen Rechner angeordneten Datenkapsel und der Änderung des Zugriffscodes und der erneuten Abspeicherung mit dem geänderten Zugriffscode im Netz,

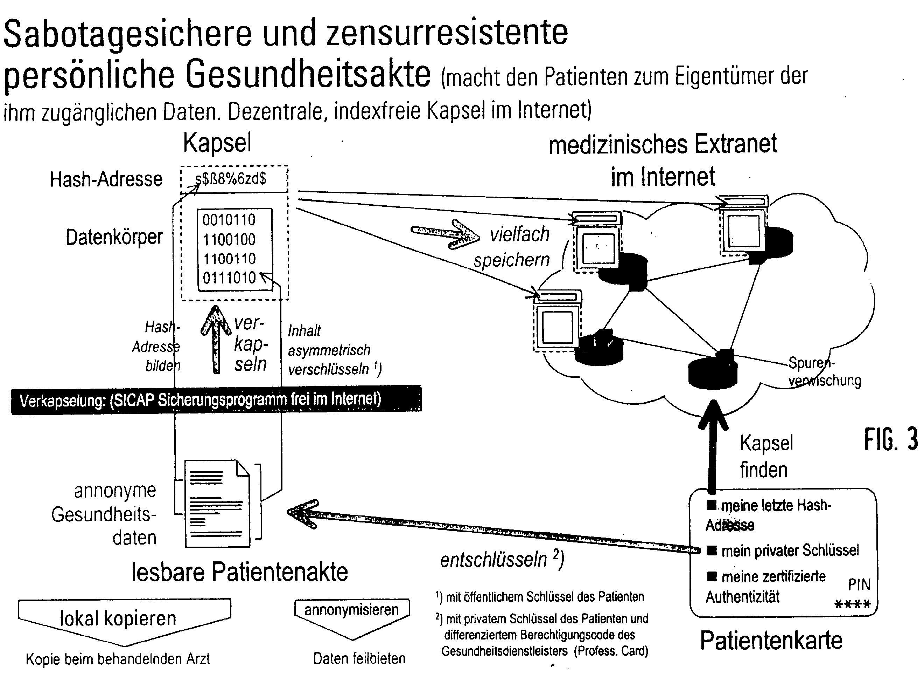

- Fig. 3: eine schematische Darstellung der Organisation einer erfindungsgemäßen gesicherten persönlichen Gesundheitsakte im Internet,
- Fig. 4: eine Darstellung der persönlichen Gesundheitsakte für die private Bearbeitung durch den Patienten,
- Fig. 5: eine der Fig. 4 entsprechende Darstellung der Bearbeitungsmöglichkeiten der persönlichen Gesundheitsakte durch den Arzt,
- Fig. 6: eine Darstellung der Dokumententypen der Gesundheitsakte mit einem Beispiel für die Aufteilung der Informationen auf verschiedene Kapseln mit unterschiedlichen Hash-Adressen,
- Fig. 7: den Ablauf einer Behandlung, Überweisung und Rezeptstellung mit Karte und Patientenakte im Internet unter Verwendung einer erfindungsgemäßen gesicherten Gesundheitsakte und
- Fig. 8: Aufbau und Organisation einer persönlichen Zugangskarte zur internetbasierten erfindungsgemäßen Gesundheitsakte.

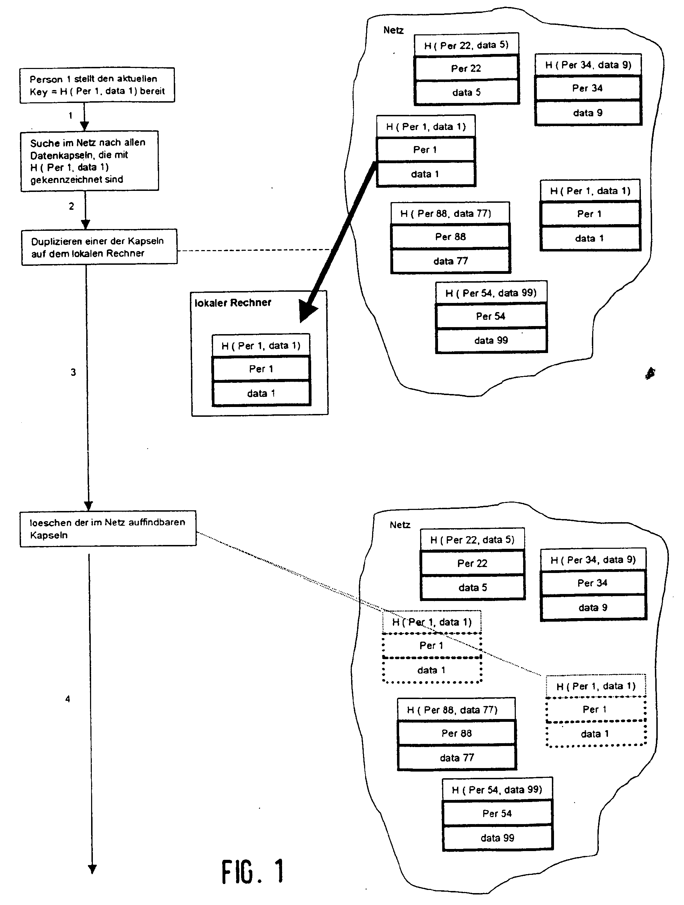

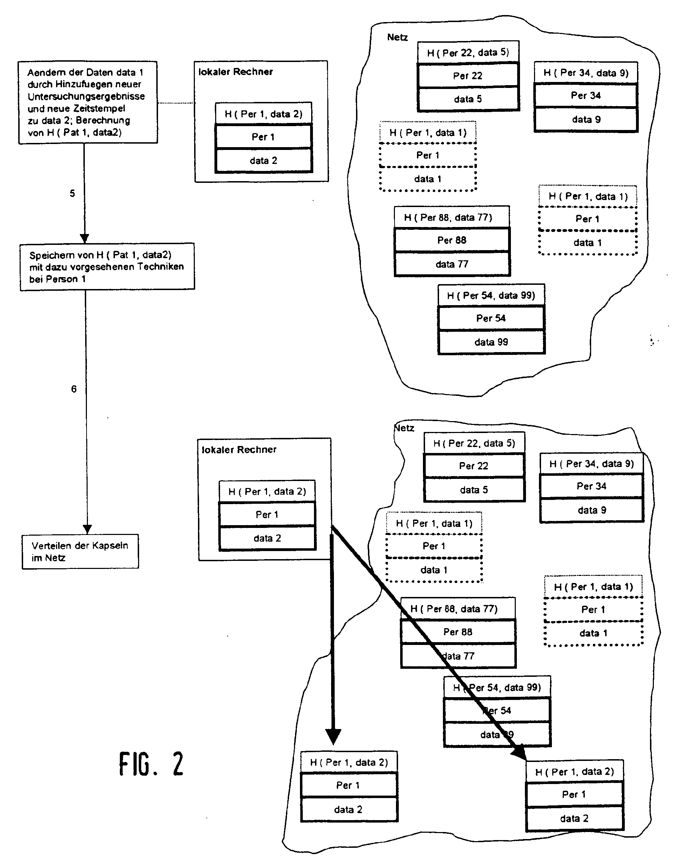

In Fig. 1 ist anhand eines schematischen Ablaufdiagramms dargestellt, wie zunächst eine Person 1 einen aktuellen Zugriffscode einen Key H bereitstellt, der aus persönlichen Daten und Speicherdaten, genannt data 1, gebildet ist. Mit diesem Key kann nach allen Datenkapseln gesucht werden, die mit dem entsprechenden Key im Netz gespeichert sind. Wird eine solche Datenkapsel gefunden - unter einer Datenkapsel versteht man eine Mehrzahl durch einen gemeinsamen Zugriffscode gesicherter Patientendaten in einer besonderen Datenstruktur entsprechend den Anforderungen des jeweiligen Speicher-Netzes - so wird eine Kopie dieser Datenkapsel auf dem lokalen Rechner gezogen und - bei Vorliegen einer Änderungsberechtigung, die Teil des aktuellen Keys ist und auf diesem nicht lesbar enthalten sein soll, alle im Netz auffindbaren entsprechenden Datenkapseln gelöscht. Dieses Löschen der Datenkapseln ist in Fig. 1 rechts unten durch die Strichpunktierung der zwei vorhandenen Datenkapselkopien im Netz dargestellt. Die Fig. 2 zeigt, wie durch Ändern der Daten data 1 durch Hinzufügen neuer Untersuchungsergebnisse oder einem neuen Zeitstempel eine Veränderung zu data 2 und damit eine Änderung des Zugriffscodes automatisch bewerkstelligt wird. Mit diesem geänderten Zugriffscode wird die auf dem lokalen Rechner angeordneten nunmehr geänderte Datenkapsel mit den üblichen Techniken wieder gespeichert und im Netz verteilt. Man erkennt dies rechts unten in Fig. 2, wo nunmehr zwei geänderte Datenkapseln mit dem Zugriffscode H (Per 1, data 2) gespeichert sind, während nach wie vor die alten Datenkapseln mit dem Zugriffscode H (Per 1, data 1) gelöscht sind.

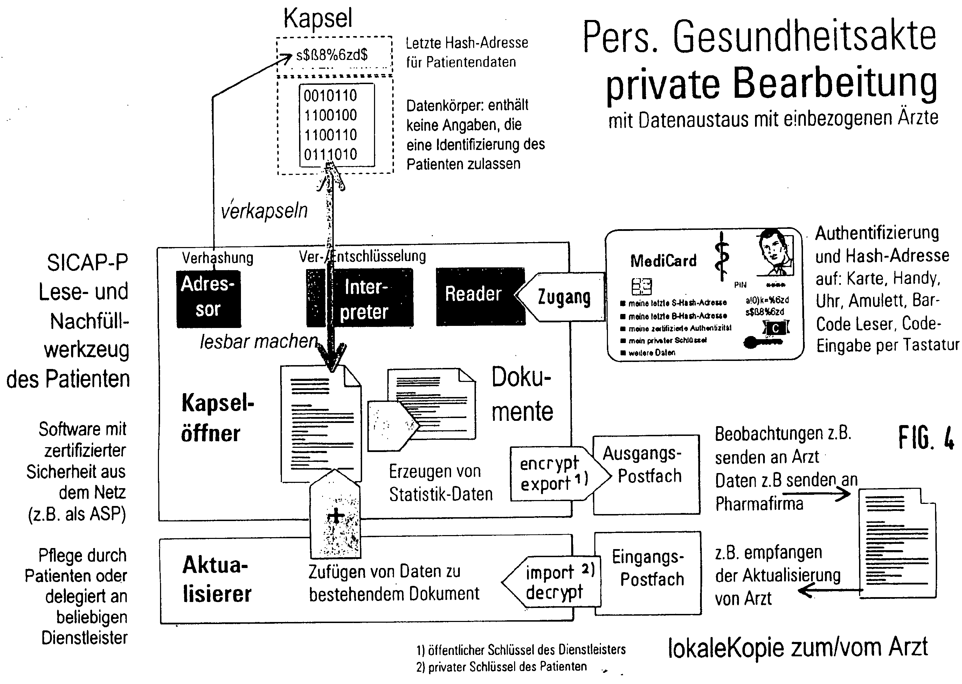

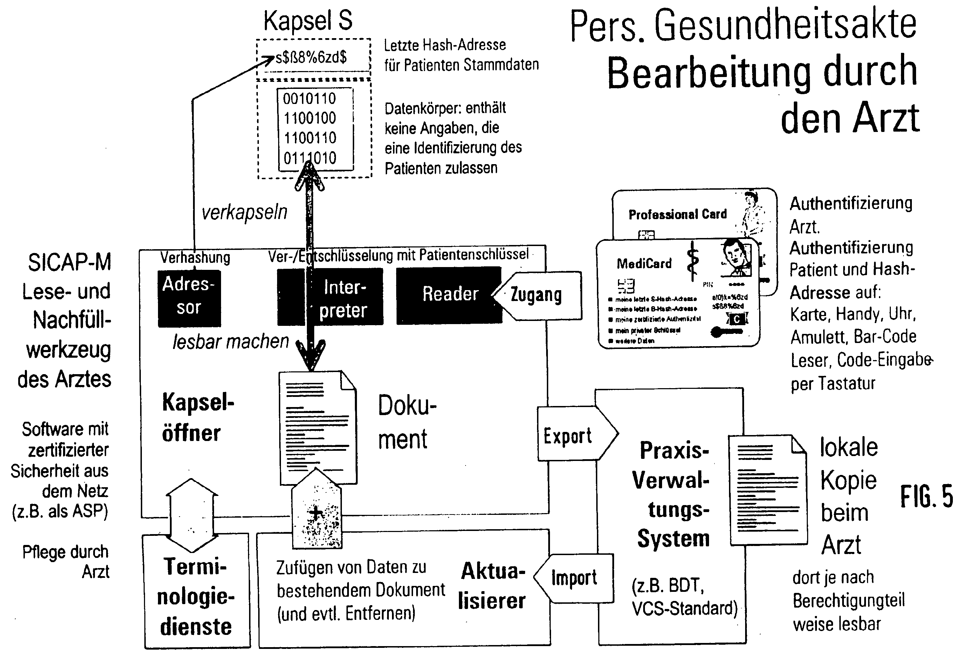

Die Fig. 1 zeigt schematisch den Aufbau einer sabotagesicheren und zensurresistenten persönlichen Gesundheitsakte, die den Patienten zum Eigentümer der ihm zugänglichen Daten macht, wobei die Gesundheitsakte eine oder mehrere dezentrale indexfreie Kapseln im Internet umfasst. In den Fig. 4 und 5 sind die verschiedenen Möglichkeiten des Einspeicherns und Auslesens in oder aus der im Internet gespeicherten Gesundheitsakte einmal für den Patienten selbst und einmal als Ausführungsbeispiel eines zugelassenen Nutzers durch den Arzt dargestellt, wobei die Authentifizierung und die Hash-Adresse, die grundsätzlich auf verschiedenartigen Zugangsgeräten angeordnet sein kann, wie beispielsweise einem Handy, einer Uhr, einem Amulett, einem elektronischen Etikett in Form eines Transponders, einem Barcodeleser oder durch Codeeingabe per Tastatur, wie im gezeigten Ausführungsbeispiel anhand einer Chipcard realisiert ist, die in ihrem Aufbau und in ihrer Datenorganisation und noch etwas genauer dargestellt ist. So kann die persönliche Gesundheitsakte vom Arzt gemäß Fig. 5 wie folgt verwendet werden:

Der Patient, körperlich anwesend, überlässt dem Arzt physikalische persönliche Patientenkarte, Arzt findet Kapsel(n) im Internet und öffnet sie mit Patientenkarte (und Arztkarte). Er trägt Behandlungstatsache und Behandlungstermin ein, macht eine lokale Kopie und verkapselt neu mit (zum Beispiel ihm bekannter oder unbekannter) neuer letzter Hash-Adresse und setzt die neue Kapsel wieder ins Internet ab. Wenn sich dabei die Hash-Adresse geändert hat, werden alle alten Kapseln durch Ausführung entsprechend vorzusehender Programmteile gelöscht. Der Arzt arbeitet ab jetzt bis zu einem wichtigen Zwischenabschluss auf seiner lokalen Kopie und verwendet diese für Überweisungen und Teledienste. Der Patient kann sich mittels Authentifikation im Netz ausweisen. Der Nachtrag der Behandlungsergebnisse auf der Patientenkarte muss separat erfolgen. Bei asymmetrischem Schlüssel auch ohne Patientenkarte möglich, solange ihm gültige Hash-Adresse benannt und nicht verändert wird.

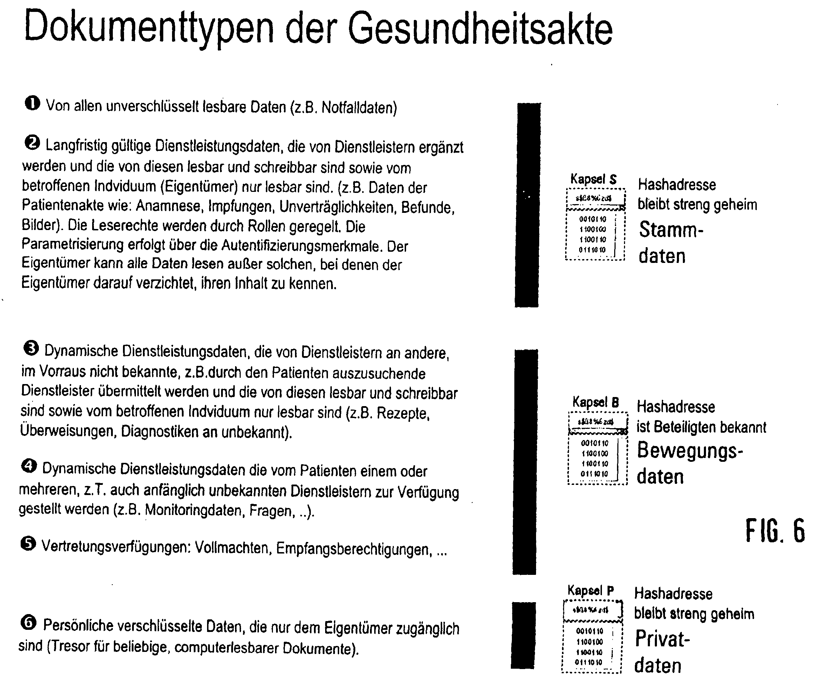

In Fig. 6 sind die verschiedenen Dokumententypen der Gesundheitsakte nach Art ihrer Ermittlung und ihrer Bedeutung für die Gesundheitsakte und auch im Hinblick auf die unterschiedlich hohen Verschlüsselungs- und unterschiedlichen Zugangsmöglichkeiten angedeutet. Speziell die in der sogenannten Kapsel B gespeicherten Patientendaten - auch hier könnte es sich selbstverständlich wieder um mehrere unterschiedliche Datenkapseln handeln - betreffen Daten, die weniger geheimhaltungsbedürftig sind und zu denen beispielsweise auch sogenannte Statistikdaten gehören, die von entsprechenden Dienstleistern (gegen entsprechende Vergütung an den Patienten) jederzeit abrufbar sind.

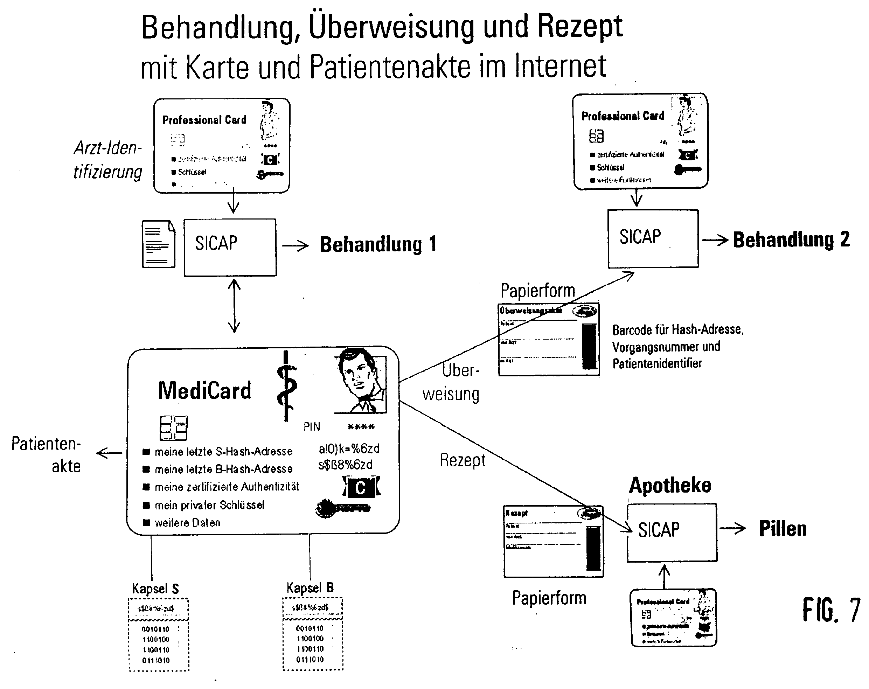

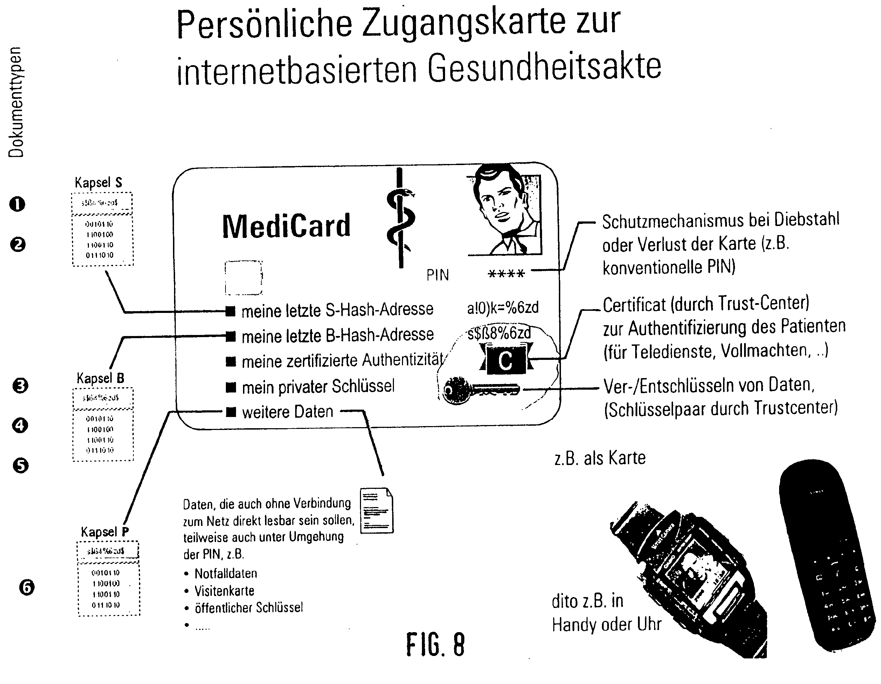

Der Weg bei einer Behandlung, Überweisung oder Rezeptstellung mithilfe von Chipkarten als Zugangskarten zur elektronischen persönlichen Gesundheitsakte im Internet sind in Fig. 7 schematisch als Diagramm angedeutet, während - wie bereits angesprochen - die Fig. 8 eine Chipkarte als persönliche Zugangskarte des Patienten zu seiner elektronisch gespeicherten Gesundheitsakte anhand der grafisch angedeuteten verschiedenen Zugriffsmöglichkeiten näher erläutert.

Um die persönliche Gesundheitsakte für Telemedizin zu verwenden, arbeitet der Arzt zum Beispiel mit den Daten aus seiner lokalen Kopie und mit der von ihm bevorzugten Technik und verwendet diese für die Teledienste. Der Patient kann sich mittels seiner Authentifikation im Netz ausweisen und somit mit Berechtigung an Telediensten teilnehmen.

Die persönliche Patientenakte kann weitere Bereiche besitzen, in die Daten geschrieben werden können und aus denen Daten gelesen werden können, wobei diese Bereiche für die Hash-Bildung ausgespart werden, sodass Dateneintragungen in diese Bereiche zu keiner Veränderung der Hash-Adresse führen. Diese Bereiche können auch für privates Gesundheitsmanagement verwendet werden, sodass hier Messwerte aus Geräten und Daten von Labels von Medikamenten und Heil- und Hilfsmitteln eingetragen werden.

## Patentansprüche

1. Gesicherte elektronische Gesundheitsakte zur Verwaltung aller gesundheitsrelevanten Daten, einschließlich früherer Diagnosen und Behandlungen, eines Patienten in Form von Datenkapseln auf mehreren dezentralen Servern eines Netzes mit einem vom Patienten freigebbaren Zugriffscode, **dadurch gekennzeichnet, dass** bei jeder Änderung oder Ergänzung einer aufgerufenen Datenkapsel die alten Datenkapsel im Netz gelöscht werden und ein neuer Zugriffscode gebildet wird, unter dem die geänderte Datenkapsel wieder neu im Netz gespeichert wird.

2. Gesundheitsakte nach Anspruch 1, **dadurch gekennzeichnet, dass** der Zugriffscode aus persönlichen Daten und Speicherdaten nach Art eines Hash-Keys ausgebildet ist.

3. Gesundheitsakte nach Anspruch 1 oder 2, **dadurch gekennzeichnet, dass** der Zugriffscode eine speziell gesicherte Änderungsberechtigung enthält, über die das automatische Löschen der alten Datenkapseln bewirkt wird.

4. Gesundheitsakte nach einem der Ansprüche 1 bis 3, **dadurch gekennzeichnet, dass** die Datenkapseln in einem zensurresistenten Extra-Netz ("freenet") gespeichert sind.

5. Gesundheitsakte nach Anspruch 4, **dadurch gekennzeichnet, dass** das Extra-Netz so ausgebildet ist, dass die Daten-Kapseln selbstorganisiert an unterschiedliche Server weitervermittelt und mehrfach identisch abgespeichert werden, sodass sich dabei möglicherweise auftretende Spuren verlieren und nicht zurückverfolgbar sind.

6. Gesundheitsakte nach Anspruch 5, **dadurch gekennzeichnet, dass** der Patient durch Parameterisierung eines Zählers die Zahl der identischen Sicherheitskopien bestimmen kann.

7. Gesundheitsakte nach einem der Ansprüche 1 bis 6, **dadurch gekennzeichnet, dass** die Daten verschlüsselt abgespeichert sind.

8. Gesundheitsakte nach Anspruch 7, **gekennzeichnet durch** die Verwendung asymmetrischer Schlüssel.

9. Gesundheitsakte nach Anspruch 8, **dadurch gekennzeichnet, dass** der private Schlüssel oder das Schlüsselpaar ein Bestandteil der persönlichen Berechtigungsinformation für das Lesen des Inhalts auf dem persönlichen Teil einer gespeicherten Datenkapsel ist.

10. Gesundheitsakte nach einem der Ansprüche 1 bis 9, **dadurch gekennzeichnet, dass** die Inhalte der Datenkapseln durch spezielle Unter-Zugriffscodes von entsprechend autorisierten Dritten, zum Beispiel Ärzten, Dienstleistern, Pharmafirmen oder dergleichen in begrenztem Umfang lesbar sind.

11. Gesundheitsakte nach Anspruch 10, **dadurch gekennzeichnet, dass** Zugangseinrichtungen vorgesehen sind, die es ermöglichen, bestimmte Teile der Daten als Statistik-Daten zu extrahieren, zu ergänzen, zu kombinieren und zu schematisieren.

12. Gesundheitsakte nach Anspruch 11, **dadurch gekennzeichnet, dass** die anonymisierten Statistikdaten in eine spezielle Statistik-Kapsel eingegeben und gespeichert werden, die mit einer global geltenden Kapsel-Adresse versehen ist.

13. Gesundheitsakte nach einem der Ansprüche 1 bis 12, **dadurch gekennzeichnet, dass** die Zugriffscodes in speziellen, vorzugsweise tragbaren Zugangsgeräten (wie zum Beispiel Karte, Handy, Uhr, Amulett oder dergleichen) implementiert sind, die ihrerseits durch ein Authentifizierungssystem gesichert sind.

14. Gesundheitsakte nach einem der Ansprüche 1 bis 13, **dadurch gekennzeichnet, dass** zumindest Teile der Patientenakten in Ablagevorrichtungen bei Ärzten, Dienstleistern oder dergleichen gespeichert sind, die für den Patienten zugänglich sind (und im Falle des Verlustes einer Kapsel-Adresse eine Rekonstruktion einer neuen Datenkapsel aus diesen Kopien ermöglichen).
